# EUROPEAN PATENT APPLICATION

(11) **EP 4 582 136 A1**
(43) Date of publication of application: **09.07.2025**
(21) Application number: 23859375.0
(22) Date of filing: 30.08.2023
(51) Int. Cl.: A61N 1/36

(54) **PULSE GENERATOR, ASSEMBLING METHOD THEREFOR, AND STIMULATION SYSTEM THEREOF**

(30) Priority: 01.09.2022 CN 202211065837
(71) Applicant: Sceneray Co., Ltd., Suzhou, Jiangsu 215123 (CN)
(72) Inventor: XU, Zhoudong, Suzhou, Jiangsu 215123 (CN); WU, Guoliang, Suzhou, Jiangsu 215123 (CN); ZHU, Weiran, Suzhou, Jiangsu 215123 (CN); ZHANG, Haitao, Suzhou, Jiangsu 215123 (CN); LI, Zheng, Suzhou, Jiangsu 215123 (CN); LIU, Bin, Suzhou, Jiangsu 215123 (CN); XIA, Ning, Suzhou, Jiangsu 215123 (CN); YUAN, Zhiping, Suzhou, Jiangsu 215123 (CN)
(74) Representative: Cabinet Beaumont
(86) International application number: PCT/CN2023/115708
(87) International publication number: WO 2024/046346

(57) **Abstract**

Provided is a pulse generator, an assembling method for a pulse generator, and a stimulation system. The pulse generator includes a connection module (1), a generator module (2) and a battery module (3). The connection module (1) includes a top cover (11) and a channel assembly (12). At least part of the channel assembly (12) is disposed in the top cover (11) and is configured for insertion of an insertion member. The generator module (2) includes a first housing (25), a circuit board (23) and a battery connection assembly. The connection module (1) is mounted on the generator module (2), and the circuit board (23) is disposed in the first housing (25) and is electrically connected to the channel assembly (12). The battery module (3) includes a second housing (33) and a battery (34) located in the second housing (33). The battery module (3) is mounted on the generator module (2) and is located outside the generator module (2), and the battery (34) is electrically connected to the circuit board (23) through the battery connection assembly.

## Description

The present application claims priority to Chinese Patent Application No. 202211065837.5, filed with the China National Intellectual Property Administration (CNIPA) on Sep. 01, 2022, the disclosure of which is incorporated herein by reference in its entirety.

### TECHNICAL FIELD

The present application relates to the technical field of implantable medical devices, for example, a pulse generator, an assembling method for a pulse generator, and a stimulation system.

### BACKGROUND

Neural electrical stimulation is a commonly used means of treating nerve dysfunction and promoting the rehabilitation of nerve injuries, and the neural electrical stimulation system applies electrical stimulation to relevant parts through electrodes and a pulse generator that are implanted in the body. The assembling process of the implantable pulse generator is relatively complex, the components of the pulse generator are connected in a stacking manner, during assembly, the next step can be performed only after the previous step is completed, and multiple different procedures cannot be performed simultaneously, so that the assembling period of a product is relatively long. Before the product is assembled and leaves the factory, when a failure occurs in the product, the product cannot be used, and the product can only be used after being completely disassembled and re-assembled, causing consumption of a large quantity of raw materials and labor costs and making it difficult to control the cost.

### SUMMARY

The present application provides a pulse generator, an assembling method for a pulse generator, and a stimulation system to solve the above-described problems.

The present application provides a pulse generator. The pulse generator includes a connection module, a generator module and a battery module. The connection module includes a top cover and a channel assembly, and at least part of the channel assembly is disposed in the top cover and is configured for insertion of an insertion member. The generator module includes a first housing, a circuit board and a battery connection assembly. The connection module is mounted on the generator module, and the circuit board is disposed in the first housing and is electrically connected to the channel assembly. The battery module includes a second housing and a battery located in the second housing. The battery module is mounted on the generator module and is located outside the generator module, and the battery is electrically connected to the circuit board through the battery connection assembly.

In an embodiment, the top cover is an insulation member, the top cover includes a front cover and a rear cover, the front cover and the rear cover are engaged to form an accommodation cavity, and at least one first channel groove is disposed on an inner side of the rear cover facing the front cover. The channel assembly includes a channel cover and multiple connection members, a second channel groove corresponding to a first channel groove of the at least one first channel groove is formed on a side of the channel cover facing the rear cover, after the channel cover and the rear cover are engaged, the first channel groove and the second channel groove form an axial channel for insertion of the insertion member, multiple radial grooves are distributed on the axial channel at intervals, one connection member of the multiple connection members which is configured to be electrically connected to the insertion member is disposed in each radial groove of the multiple radial grooves, and the multiple connection members are electrically connected to the circuit board.

In an embodiment, a first metal layer and a through hole communicating the inside and outside of the channel cover are disposed on an inner wall of each radial groove of the multiple radial grooves, an outer surface of the channel cover is provided with multiple second metal layers, each second metal layer of the multiple second metal layers is electrically connected to the first metal layer in a radial groove corresponding to one through hole among the multiple radial grooves through the one through hole, and the multiple connection members are electrically connected to the circuit board through first metal layers and the multiple second metal layers.

In an embodiment, the generator module further includes a feedthrough member, the feedthrough member is an insulation member, multiple third metal layers are disposed on the feedthrough member, and each third metal layer of the multiple third metal layers is electrically connected to one second metal layer of the multiple second metal layers and the circuit board.

In an embodiment, the connection module further includes a charging coil and an antenna, the charging coil is embedded in the front cover or the rear cover, the antenna is disposed on a side of the channel cover facing away from the multiple radial groove, a fourth metal layer and two fifth metal layers are disposed on the feedthrough member, the fourth metal layer is electrically connected to the antenna and the circuit board, the two fifth metal layers are electrically connected to the charging coil and the circuit board, respectively, and the fourth metal layer and the two fifth metal layers are close to two opposite ends of the feedthrough member, respectively.

In an embodiment, a first clamping portion facing the top cover is disposed on the first housing, a second clamping portion is disposed on the front cover or the rear cover, and when the connection module is mounted on the generator module, the first clamping portion is in clamping connection with the second clamping portion.

In an embodiment, a clamping groove is disposed on the first clamping portion, the second clamping portion is a protrusion which has an inclined surface and disposed on an inner surface of the rear cover, and when the connection module is mounted on the generator module, the first clamping portion is clamped on the second clamping portion under a guiding action of the inclined surface of the second clamping portion.

In an embodiment, the front cover is provided with an aperture communicating the inside and outside of the accommodation cavity, and the aperture of the front cover is a notch located at an edge of the front cover or an aperture not extending to an edge of the front cover. A first auxiliary positioning portion is disposed on an edge of the rear cover facing the first housing, a second auxiliary positioning portion is disposed on the first housing and is engaged with the rear cover, and when the connection module is mounted on the generator module, the first auxiliary positioning portion is engaged with the second auxiliary positioning portion.

In an embodiment, the first housing is provided with a tongue plate deepening into the accommodation cavity, the accommodation cavity is provided with an insulating encapsulation material for filling a gap in the accommodation cavity, the insulating encapsulation material blocks the aperture on the front cover, and the insulating encapsulation material wraps the tongue plate.

In an embodiment, the passage assembly further includes a waterproof member and a positioning member, the waterproof member and the positioning member are sequentially disposed at an opening of an axial channel from outside to inside, each of the waterproof member and the positioning member is provided with a hole which is used for the insertion member to pass through and enter the axial channel, the positioning member is configured to fix the insertion member, and the multiple connection members are each a coil spring.

In an embodiment, the connection module further includes an identification member, the identification member is disposed in the top cover, the identification member is a metal member and is a hollow structure, and the hollow structure of the identification member forms an identification of the identification member.

In an embodiment, a thread hole is disposed on the top cover.

In an embodiment, the battery connection assembly includes a positive connector and a negative connector, the battery module further includes a positive terminal and a negative terminal, the positive terminal and the negative terminal are connected to the battery, respectively, the positive terminal is electrically connected to the positive connector, and the negative terminal is electrically connected to the negative connector. The generator module further includes a temperature measuring device, and the temperature measuring device is electrically connected to the circuit board and is configured to measure a temperature of the battery.

In an embodiment, the first housing and the second housing are titanium shells and are welded together to form a sealed accommodation space, and the second housing also serves as a housing of the battery itself.

The present application further provides an assembling method for a pulse generator. The pulse generator is a pulse generator according to any one of the foregoing. The assembling method includes that: the connection module, the generator module and the battery module are assembled separately, the first housing and the second housing are assembled and connected so that the battery module is mounted on the generator module, and the connection module is mounted on the generator module.

In an embodiment, the assembling method further includes that: the first housing and the second housing are assembled and connected through welding; and an insulating encapsulation material is injected into the top cover to connect the top cover and the first housing.

In an embodiment, the insulating encapsulation material is epoxy resin.

The present application further provides a stimulation system. The stimulation system includes a stimulation electrode, the pulse generator according to any one of the foregoing, and a wire. The wire is provided with an insertion member, the insertion member is configured to be inserted into the channel assembly, and the pulse generator is electrically connected to the stimulation electrode through the wire.

In an embodiment, the stimulation system further includes a converter. Multiple stimulation electrodes are provided. The converter is electrically connected to the pulse generator and is configured to divide a stimulation pulse signal of the pulse generator into multiple channels of stimulation pulse signals, and each channel of stimulation pulse signal of the multiple channels of stimulation pulse signals is released through one stimulation electrode of the multiple stimulation electrodes.

In an embodiment, the wire includes an extension wire and multiple electrode wires, the converter is electrically connected to the pulse generator through the extension wire, and each stimulation electrode of the multiple stimulation electrodes is electrically connected to the converter through the insertion member on one electrode wire of the multiple electrode wires.

### BRIEF DESCRIPTION OF DRAWINGS

FIG. 1 is a schematic structural diagram of a pulse generator according to an embodiment of the present application;
FIG. 2 is a schematic structural diagram of a pulse generator with a front cover removed according to an embodiment of the present application;
FIG. 3 is a schematic structural diagram of a pulse generator with a front cover and a first housing removed according to an embodiment of the present application;
FIG. 4 is a schematic exploded view of a pulse generator according to an embodiment of the present application;
FIG. 5 is a schematic structural diagram of a connection module according to an embodiment of the present application;
FIG. 6 is a sectional view of a connection module according to an embodiment of the present application;
FIG. 7 is a schematic exploded view of a generator module according to an embodiment of the present application;
FIG. 8 is a schematic exploded view of a connection module according to an embodiment of the present application;
FIG. 9 is a schematic structural diagram of a pulse generator according to an embodiment of the present application;
FIG. 10 is a schematic structural diagram of a stimulation system according to an embodiment of the present application; and
FIG. 11 is a flowchart of an assembling method according to an embodiment of the present application

### List of reference numbers

- 1: connection module
- 11: top cover
- 111: front cover
- 112: rear cover
- 1121: first auxiliary positioning portion
- 113: first channel groove
- 114: channel cover
- 115: second channel groove
- 116: axial channel
- 117: radial groove
- 118: first metal layer
- 119: second metal layer
- 120: through hole
- 12: channel assembly
- 121: waterproof member
- 122: positioning member
- 13: charging coil
- 131: charging terminal
- 14: antenna
- 15: second clamping portion
- 16: aperture
- 17: connection member
- 18: identification member
- 19: thread hole
- 2: generator module
- 21: feedthrough member
- 221: fourth metal layer
- 222: fifth metal layer
- 22: third metal layer
- 23: circuit board
- 24: temperature measuring device
- 25: first housing
- 251: first clamping portion
- 252: tongue plate
- 253: second auxiliary positioning portion
- 3: battery module
- 31: positive connector
- 32: negative connector
- 33: second housing
- 34: battery
- 41: extension wire
- 42: electrode wire
- 43: stimulation electrode
- 44: pulse generator
- 45: converter
- 46: insertion member

### DETAILED DESCRIPTION

Example embodiments will now be described with reference to the accompanying drawings. However, the example embodiments are capable of being implemented in various forms and should not be construed as limited to the embodiments set forth herein; rather, these embodiments are provided to make the present application more comprehensive and to convey the concepts of the example embodiments to those skilled in the art. Like reference numerals refer to the same or similar structures in the accompanying drawings; therefore, repeated descriptions thereof will be omitted.

The words for expressing positions and directions described in the present application are described by using the accompanying drawings as an example, but may also be changed as required, and the changes are included in the scope of protection of the present application.

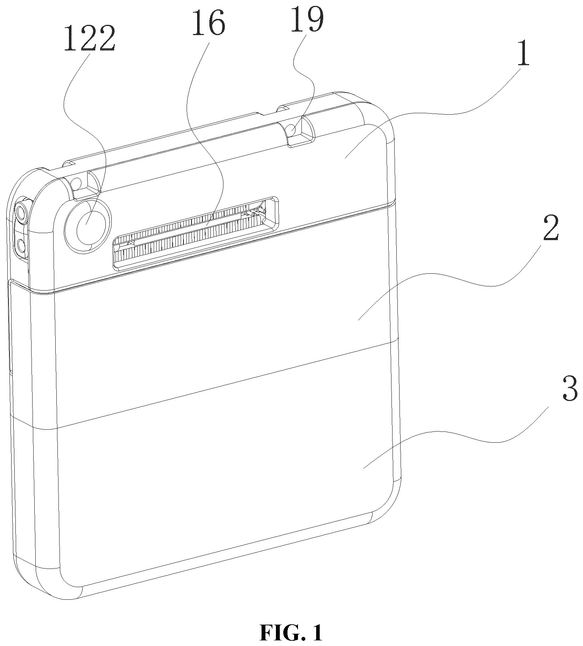

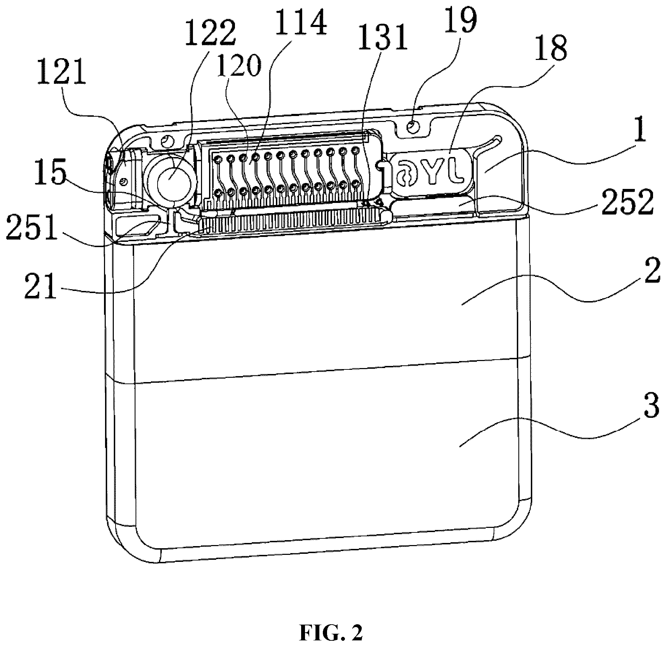

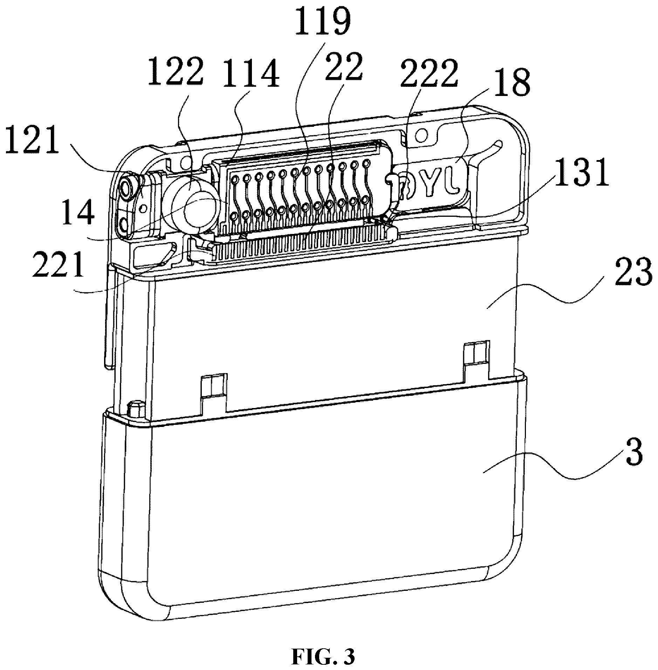

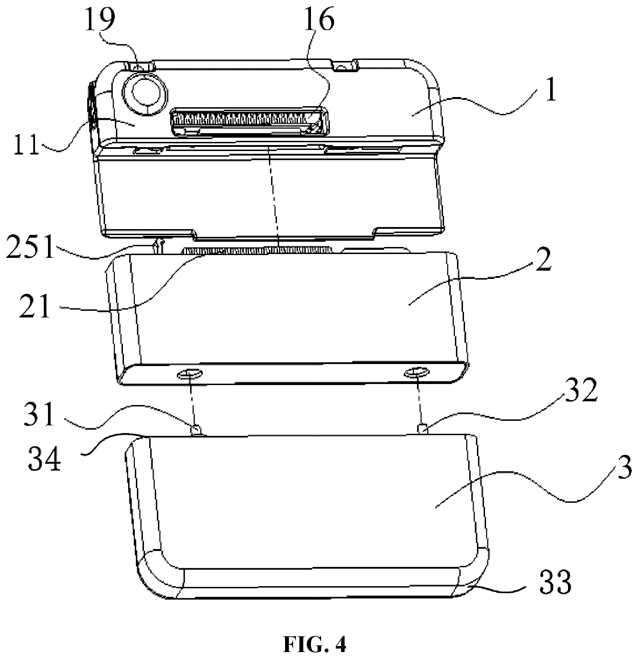

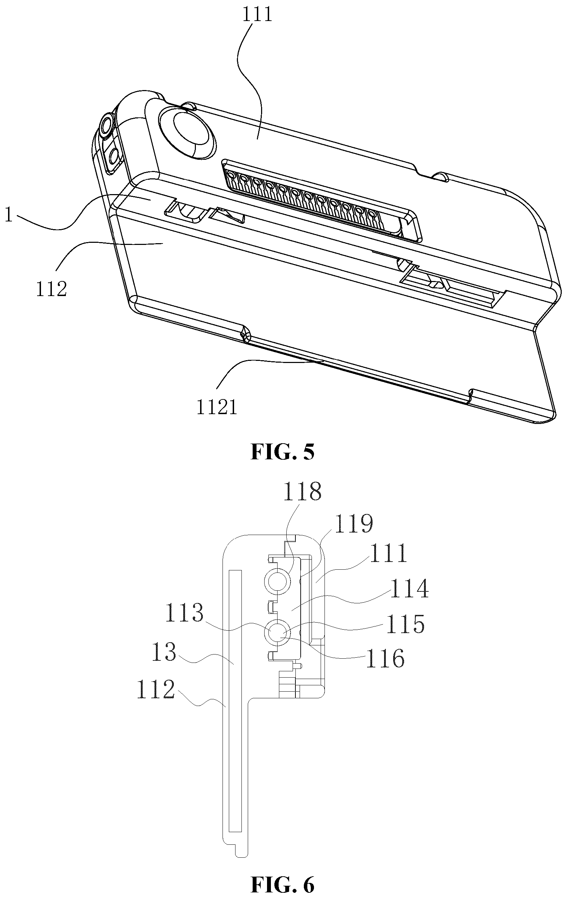

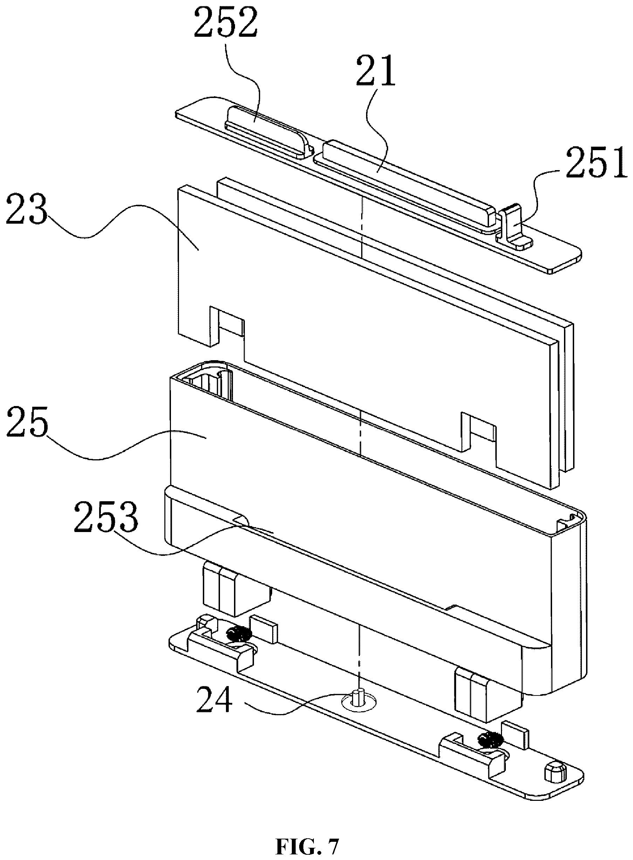

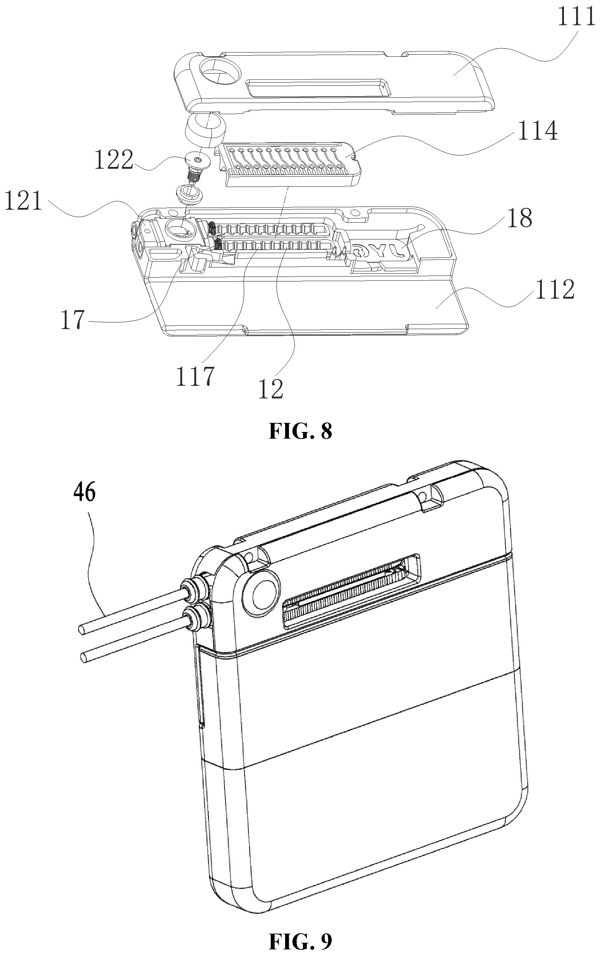

Referring to FIGS. 1 to 8, the present application provides a pulse generator. The pulse generator includes a connection module 1, a generator module 2 and a battery module 3.

The connection module 1 includes a top cover 11 and a channel assembly 12. At least part of the channel assembly 12 is disposed in the top cover 11 and is configured for insertion of an insertion member, and the insertion member is, for example, an insertion end of an extension wire 41. The generator module 2 includes a first housing 25, a circuit board 23 and a battery connection assembly. The connection module 1 is mounted on the generator module 2, and the circuit board 23 is disposed in the first housing 25 and is electrically connected to the channel assembly 12. The battery module 3 includes a second housing 33 and a battery 34 located in the second housing 33, and the second housing 33 also serves as a housing of the battery 34 itself, in other words, the second housing 33 is the housing of the battery 34 itself, so that there is no need to provide one housing outside the housing of the battery 34, thereby simplifying the structure of the product and saving costs. The battery module 3 is mounted on the generator module 2 and is located outside the generator module 2, and the battery 34 is electrically connected to the circuit board 23 through the battery connection assembly.

The pulse generator is designed to include the connection module 1, the generator module 2 and the battery module 3 which are independent of each other, the three mutually independent modules may be assembled separately during their respective assembly procedures in a multi-threaded manner, so that the overall assembly period of the pulse generator is shortened. Moreover, when the assembled pulse generator is damaged, the damaged module may be replaced without requiring the whole pulse generator to be discarded, thereby reducing the production cost of enterprises.

The assembly of the connection module 1, the generator module 2 and the battery module 3 includes two steps. The first step is to assemble each module itself, the generator module 2 is used as an example, when the generator module 2 is assembled itself, the circuit board 23 and the battery connection assembly are sequentially mounted and fixed in the second housing 33 to complete the preliminary assembly. The second step is to butt and assemble the generator module 2 with the assembled connection module 1, and then assemble the bottom with the assembled battery module 3, thereby completing the assembly of the pulse generator. After the assembly is completed, when any of the assembly modules malfunctions or is damaged during detection, the damaged module may be removed and replaced with a module of the same type for reassembly, whereby the cost can be saved. In addition, when the relatively independent multiple modules are assembled, the modules may be assembled independently and simultaneously at multiple workstations without interfering with each other, so that the high working efficiency can be ensured and the production period of each pulse generator can be shortened. In addition, the connection module 1, the generator module 2 and the battery module 3 in a modular design may each be designed into different models. Modules of different models adopt standard or unified interfaces, for example, the battery module 3 may be made into modules with different battery capacities to match a variety of power requirements, for example, the battery module 3 may be configured with a small-capacity battery, a large-capacity battery, a round battery, or a square battery, and the like.

In an embodiment, the top cover 11 is an insulation member and may be made of plastic material. The top cover 11 includes a front cover 111 and a rear cover 112. The front cover 111 and the rear cover 112 may be two insulation housings with matching shapes. The front cover 111 and the rear cover 112 are engaged to form an accommodation cavity. At least one first channel groove 113 is disposed on an inner side of the rear cover 112 facing the front cover 111.

The channel assembly 12 includes a channel cover 114 and multiple connection members 17. A second channel groove 115 corresponding to the first channel groove 113 is disposed on a side of the channel cover 114 facing the rear cover 112. After the channel cover 114 and the rear cover 112 are engaged, the first channel groove 113 and the second channel groove 115 form an axial channel 116 for insertion of the insertion member. The first channel groove 113 and the second channel groove 115 may be each half of the axial channel 116. Multiple radial grooves 117 are distributed on the axial channel 116 at intervals, one connection member 17 electrically connected to the insertion member is disposed in each radial groove 117, and the radial groove 117 communicates with the axial channel 116 internally. The connection member 17 is, for example, a coil spring and a conductive wire and is electrically connected to the circuit board 23. The first channel groove 113 is formed on the rear cover 112, the second channel groove 115 is formed on the channel cover 114, the first channel groove 113 and the second channel groove 115 form one axial channel 116 through buckling of the channel cover 114 and the rear cover 112 during connection, and the connection member 17 is connected in the axial channel 116. Compared with an assembly manner in which two channel covers 114 are separately disposed between the rear cover 112 and the front cover 111, and the two channel covers 114 are engaged to form the axial channel 116, in this embodiment, the first channel groove 113 is formed on the rear cover 112, and the first channel groove 113, as part of the axial channel 116, cooperates with the second channel groove 115 to jointly form the axial channel 116. This manner is more integrated and makes full use of the structure of the rear cover 112 to reduce its thickness, the assembly structure is prefabricated on the rear cover 112, so that the dimensional fit tolerances when multiple components of the top cover 11 are assembled can be significantly reduced, and thus, the overall volume of the pulse generator is decreased, thereby making it more lightweight.

A bottom of the rear cover 112 may also have a section of strip-shaped protrusion which is configured to fit with a groove corresponding to the bottom of the rear cover 112 and located on the generator module 2. The fixing effect is achieved by filling sealant and the like during assembly.

Exemplarily, a first metal layer 118 and a through hole 120 communicating the inside and outside of the channel cover 114 are disposed on an inner wall of the radial groove 117, an outer surface of the channel cover 114 is provided with multiple second metal layers 119, each second metal layer 119 is electrically connected to the first metal layer 118 in a radial groove corresponding to one through hole 120 among the multiple radial grooves 117 through the one through hole 120, and the multiple connection members 17 are electrically connected to the circuit board 23 through the first metal layer 118 and the second metal layer 119. The first metal layer 118 and the second metal layer 119 may be formed on the channel cover 114 by processes such as electroplating, which are not enumerated herein. During operation, the generator module 2 generates a stimulation signal, the stimulation signal is transmitted through the second metal layer 119 and passes through the through hole 120, then is transmitted through the first metal layer 118 and onto the connector 17, and finally, the connection member 17 transmits the stimulation signal to the insertion member and then to other parts to complete the signal transmission. The first metal layer 118 and the second metal layer 119 are conductive metal materials with uniform thickness. The first metal layer 118 and the second metal layer 119 are respectively attached or formed on an inner side of the channel, inside the through hole 120, and an outer side of the channel. The first metal layer 118 and the second metal layer 119 have good electrical connection stability. Moreover, the contact area when the first metal layer 118 and the second metal layer 119 are connected is relatively large. Compared with the connections between points and points and between points and surfaces, in this embodiment, a surface-to-surface connection of the metal layer has stronger stability, and in addition, the first metal layer 118 and the second metal layer 119 are not easy to fall off and move and cause the short circuit. When the pulse generator is vibrated or collided, the first metal layer 118 and the second metal layer 119 may still keep the good electrical connection effect.

In an embodiment, the generator module 2 further includes a feedthrough member 21. The feedthrough member 21 is an insulation member. Multiple third metal layers 22 are disposed on the feedthrough member 21. Each third metal layer 22 is electrically connected to one second metal layer 119 and the circuit board 23. The multiple third metal layers 22 are connected to the multiple second metal layers 119 in one-to-one correspondence. During operation, a stimulation signal generated by the generator module 2 is electrically connected to and transmitted to the connection module 1 through the multiple third metal layers 22 and the multiple second metal layers 119, then the stimulation signal is transmitted to the outside through the insertion member inserted into the connection module 1, and finally, the stimulation signal is applied to a to-be-stimulated point through a stimulation electrode.

In an embodiment, the battery connection assembly includes a positive connector 31 and a negative connector 32, the battery module 3 further includes a positive terminal and a negative terminal, the positive terminal and the negative terminal are connected to the battery 34, the positive terminal is electrically connected to the positive connector 31, and the negative terminal is electrically connected to the negative connector 32. After the battery module 3 is connected to the generator module 2, the battery module 3 is connected to a respective one of bottom interfaces of the generator module 2 through the positive connector 31 and the negative connector 32, to supply power to the generator module 2.

Exemplarily, the generator module 2 further includes a temperature measuring device 24, and the temperature measuring device 24 is electrically connected to the circuit board 23 and is configured to measure a temperature of the battery 34. The temperature measuring device 24 may extend into the battery module 3, or a concave portion is disposed on the battery module 3. A temperature measuring end of the temperature measuring device 24 contacts the concave portion on the battery module 3. The temperature measuring device 24 is, for example, a contact temperature sensor. The temperature of the battery 34 is measured by means of heat transfer during operation, the temperature measuring device 24 may also be a non-contact infrared temperature measuring device, and the temperature of the battery 34 is measured by means of the infrared temperature measuring device. The temperature information may be stored in the storage device or transmitted to an external device, thereby enabling the medical staff to promptly know the internal condition of the pulse generator.

Exemplarily, the connection module 1 further includes a charging coil 13 and an antenna 14. The charging coil 13 is embedded in the front cover 111 or the rear cover 112. During charging, an external wireless charger and the charging coil 13 induce each other to generate an induced current. The power generated by the induced current is finally stored by the battery module 3, and the battery of the pulse generator does not need to be physically replaced by the user to maintain the pulse generator to work continuously. The induced current generated by the charging coil 13 is transmitted through a joint between the coil and the circuit board 23, and then the current is transmitted through a line on the circuit board 23, and enters the battery through the positive connector 31 and the negative connector 32 to store electricity. The wireless charging mode described above is adopted so that the service life of the pulse generator can be maintained for a long time. In addition, the charging coil 13 is embedded into the front cover 111 or the rear cover 112, on one hand, since the front cover 111 or the rear cover 112 is generally made of an inorganic insulating material, such as, a polymer material, the generation of the eddy current heat effect can be reduced or avoided, and thus the charging efficiency is high, on the other hand, after the charging coil 13 is embedded, the space occupied by the charging coil 13 can be avoided, and thus the size of the product can be reduced.

The antenna 14 is disposed on a side of the channel cover 114 facing away from the radial groove 117, a fourth metal layer 221 and two fifth metal layers 222 are disposed on the feedthrough member 21, the fourth metal layer 221 is electrically connected to the antenna 14 and the circuit board 23, the two fifth metal layers 222 are electrically connected to the charging coil 13 and the circuit board 23, respectively, and the fourth metal layer 221 and the fifth metal layer 222 are close to two opposite ends of the feedthrough member 21, respectively. The user can make the pulse generator communicate with an external receiving device by using the antenna 14, so that the internal information of the pulse generator is fed back to the external receiving device in real time, whereby the external receiving device can know working parameters of the pulse generator, such as a temperature of the battery during working, a battery charge, or temperature information inside the generator module 2, which helps the external medical staff to judge the stability of the performance of the pulse generator, and may also control the pulse generator through the antenna 14. Each of the fifth metal layer 222 and the charging coil 13 is electrically connected to a respective one of charging terminals 131 led out to the outside of the front cover 111 or the outside of the rear cover 112, so that the current of the charging coil 13 passes through the circuit board 23 and finally charges the battery module 3. The fourth metal layer 221 and the fifth metal layer 222 are close to two opposite ends of the feedthrough member 21, respectively, that is, the fourth metal layer 221 is close to one end of the feedthrough member 21, and the fifth metal layer 222 is close to the opposite end of the feedthrough member 21, so that the interference of the fifth metal layer 222 on the signal of the fourth metal layer 221 for communication can be reduced, and thus the signal transmission reliability is higher.

Exemplarily, a first clamping portion 251 facing the top cover 11 is disposed on the first housing, a second clamping portion 15 is disposed on the front cover 111 or the rear cover 112, and when the connection module 1 is mounted on the generator module 2, the first clamping portion 251 is in clamping connection with the second clamping portion 15. The first clamping portion 251 is, for example, a clamping plug with an inclined surface, the second clamping portion 15 is, for example, a clamping ring with a clamping groove, the plug is clamped in the groove of the clamping ring to form a clamping, and the front cover 111 and the rear cover 112 are clamped together through the first clamping portion 251 and the second clamping portion 15, so that the assembly mode is simple and convenient, the assembly is completed without complex procedures, the assembly steps are reduced, and the assembly period of the pulse generator is shortened.

Exemplarily, a clamping groove is disposed on the first clamping portion 251, the second clamping portion 15 is a protrusion having an inclined surface and disposed on the inner surface of the rear cover 112, and when the connection module 1 is mounted on the generator module 2, the first clamping portion 251 is clamped on the second clamping portion 15 under the guiding action of the inclined surface of the second clamping portion 15. During connection, a connection end of the connection module 1 is vertically butted with a connection end of the generator module 2, the protrusion with the inclined surface will firstly abut against the first clamping portion 251, the first clamping portion 251 is pushed to shift by a certain angle under the action of the inclined surface until the protrusion of the second clamping portion 15 moves into the groove of the first clamping portion 251, the first clamping portion 251 recovers the vertical angle, and the second clamping portion 15 is clamped and fixed, whereby during assembly, the assembly personnel can quickly and accurately assemble, and thus the overall assembly period of the pulse generator is shortened. The first clamping portion 251 and the second clamping portion 15 also have the auxiliary positioning function to prevent the connection module 1 and the generator module 2 from deviating from the preset position during assembly.

In an embodiment, the front cover 111 is provided with an aperture 16 communicating the inside and outside of the accommodation cavity. The aperture 16 may be a strip-shaped aperture. The aperture 16 of the front cover 111 may be a notch located at an edge of the front cover 111, or may be an aperture not extending to an edge of the front cover 111. The aperture 16 has two functions. The first function is that during assembly, after the connection module 1 is connected to the generator module 2, the assembly personnel may directly connect the second metal layer 119 and the third metal layer 22 through welding wires, connect the fourth metal layer 221 and the antenna 14 through welding wires, and connect the fifth metal layer 222 and the charging terminal 131 through welding wires, without requiring the assembly personnel to expose all circuit boards 23 of the connection module 1 and the generator module 2, thereby simplifying the assembly steps and reducing the rejection rate of the product. The second function is that after the welding of the second metal layer 119 and the third metal layer 22 is finished, and the second metal layer 119 and the third metal layer 22 are tested to be qualified, glue for curing may be injected into the pulse generator through the aperture 16, so that the connection module 1 and the generator module 2 are connected together, the internal components are effectively packaged, and the waterproof effect is achieved.

A first auxiliary positioning portion 1121 is disposed on an edge of the rear cover 112 facing the first housing 25. The first auxiliary positioning portion 1121 may be a protrusion of an edge of the first housing 25. A second auxiliary positioning portion 253 is disposed on the first housing 25 and is engaged with the rear cover 112. The second auxiliary positioning portion 253 may be a groove on the first housing 25, or the first auxiliary positioning portion 1121 is a groove, the second auxiliary positioning portion 253 is a protrusion on the first housing 25. When the connection module 1 is mounted on the generator module 2, the first auxiliary positioning portion 1121 and the second auxiliary positioning portion 253 are engaged, for example, the first auxiliary positioning portion 1121 is inserted into the second auxiliary positioning portion 253, or the second auxiliary positioning portion 253 is inserted into the first auxiliary positioning portion 1121, so that when the connection module 1 is mounted on the generator module 2, the first auxiliary positioning portion 1121 and the second auxiliary positioning portion 253 play a role in auxiliary positioning. Moreover, the edge of the rear cover 112 does not tilt, thereby improving the flatness of the whole pulse generator.

In an embodiment, the first housing 25 is provided with a tongue plate 252 deepening into the accommodation cavity. The accommodation cavity is provided with an insulating encapsulation material for filling a gap in the accommodation cavity. The insulating encapsulation material is, for example, epoxy resin. The insulating encapsulation material blocks the aperture 16 on the front cover 111. The insulating encapsulation material wraps the tongue plate 252. When the connection module 1 is butted with the generator module 2, and meanwhile, the tongue plate 252 may be inserted into a preset position in the accommodation cavity to play a role in guiding and auxiliary positioning for the connection of the first clamping portion 251 and the second clamping portion 15, so that the position calibration step of the first clamping portion 251 and the second clamping portion 15 during assembly is simplified, and thus the assembly is convenient. After the insulating encapsulation material is injected and cured, the insulating encapsulation material wraps the tongue plate 252, so that the connection strength of the connection module 1 and the generator module 2 can be effectively improved, the internal elements are effectively packaged, and the waterproof effect is achieved.

In an embodiment, the channel assembly 12 further includes a waterproof member 121 and a positioning member 122. The waterproof member 121 and the positioning member 122 are sequentially disposed at an opening of the axial channel 116 from outside to inside. Each of the waterproof member 121 and the positioning member 122 is provided with a hole which is used for the insertion member to pass through and enter the axial channel 116. The positioning member 122 is configured to fix the insertion member, and the connection member 17 is a coil spring. The waterproof member 121 is formed by injection molding with materials such as rubber or silica gel. During assembly, the insertion member is inserted through the axial opening and directly inserted along the axial channel 116 until the connection positions on the insertion member and all coil springs are kept in one-to-one correspondence, and then one end of the inserted insertion member is clamped and fixed in the connection module 1 by a fastener. The fastener may be a bolt threaded onto the rear cover 112. During assembly, the operator screws the bolt to make the nut press against the axial surface of the insertion member, thereby completing the fixation.

In an embodiment, the connection module 1 further includes an identification member 18. The identification member 18 is disposed in the top cover 11. The identification member 18 is a metal member and has a hollow structure. The hollow structure of the identification member 18 forms an identification of the identification member 18. The metal member may record models of modules such as the connection module 1 of the pulse generator, the generator module 2 of the pulse generator, and the battery module 3 of the pulse generator by using the hollow portion as a mark. During use, the medical staff scans the body of the user by using the X-ray machine, and may know information about various modules of the pulse generator through the identification member 18, so that the medical staff can conveniently know more information about the pulse generator.

In an embodiment, a thread hole 19 is disposed on the top cover 11. During use, the pulse generator may be threaded into the body of the user by the medical staff in a manner of making a thread pass through the aperture 16, thereby preventing the position of the pulse generator in the body from shifting due to the daily activities of the user, and reducing the failure rate of the pulse generator.

Exemplarily, the first housing 25 and the second housing 33 are titanium shells and are welded together to form a sealed accommodation space. During assembly, the step of assembling the battery module 3 at the bottom of the generator module 2 may be completed before the generator module 2 and the connection module 1 are assembled, which is not limited herein. The titanium shell is a biocompatible material, and after the titanium shell is implanted in the body of the patient, no rejection reaction occurs. Moreover, the formed sealed accommodation space can play the waterproof role.

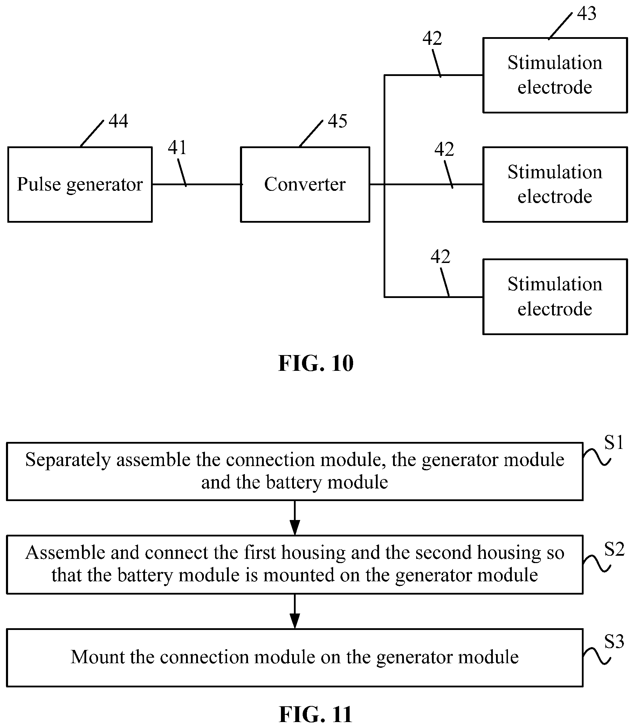

The present application further provides an assembly method for a pulse generator. The pulse generator is the pulse generator according to any one of the above embodiments. As shown in FIG. 11, the assembly method includes steps S1 to S3.

In step S1, the connection module 1, the generator module 2 and the battery module 3 are assembled separately.

Firstly, the above three modules are respectively manufactured and assembled through multiple procedures which may be performed simultaneously, and different modules may be independently completed through different production lines and procedures during assembly. After the assembly of the above three modules is completed, the quality of the product may be detected through respective detection procedures, and the modules may be processed and assembled in a multi-threaded manner. Compared with a one-line assembly mode, the better assembly efficiency is owned, and the overall assembly time of the pulse generator is shortened. Moreover, when any one of the three mutually independent modules is damaged, it is easy to replace the damaged module with a module of the same model, then, this module of the same model may be assembled with the other two modules again to form a new pulse generator.

In step S2, the first housing 25 and the second housing 33 are assembled and connected so that the battery module 3 is mounted on the generator module 2. The first housing 25 is butted with the second housing 33, so that the protruding positive connector 31 and the protruding negative connector 32 of the battery module 3 are butted at the bottom of the generator module 2, and the battery module 3 may provide the power support for the operation of the generator module 2.

In an embodiment, the step S2 includes a step S21. In step S21, the first housing 25 and the second housing are assembled and connected by welding. After the first housing 25 and the second housing 33 are connected, the first housing 25 and the second housing 33 are hermetically connected by a welding procedure.

In step S3, the connection module 1 is mounted on the generator module 2.

After the battery module 3 and the generator module 2 are connected, the generator module 2 and the connection module 1 may be snapped together in a form of a buckle, a conductive layer of the connection module 1 is electrically connected to a conductive layer of the generator module 2, and then the preliminary assembled pulse generator may be functionally tested through the testing procedure.

In an embodiment, the step S3 includes a step S31. In step S31, an insulating encapsulation material is injected into the top cover 11 to connect the top cover 11 and the first housing 25 together. The pulse generator with the qualified function will finally be filled with the insulating encapsulation material through the opening on the top cover 11, and the pulse generator is integrally formed into a sealed state after curing, so that waterproof can be achieved when the pulse generator is implanted into the body of the patient for use. The insulating encapsulation material may be epoxy resin.

The present application further provides a stimulation system. The stimulation system includes a stimulation electrode 43, the pulse generator 44 according to any one of the foregoing embodiments, and a wire. An insertion member 46 is disposed on the wire, the insertion member 46 is inserted into the channel assembly 12, and the pulse generator 44 is electrically connected to the stimulation electrode 43 through the wire. Positions of connection sites of the insertion member 46 are in one-to-one correspondence with positions of coil springs on the channel assembly 12. During operation, the electrical stimulation signal generated by the pulse generator 44 is transmitted through the channel module 12 and the insertion member 46 inserted into the channel assembly 12 and then transmitted to the stimulation electrode 43 through the wire, and the stimulation signal is released at a to-be-stimulated point of the patient through the stimulation electrode 43, to assist the patient in stimulating therapy and help the patient recover the nerve function.

In an embodiment, the stimulation system further includes a converter 45 and multiple stimulation electrodes 43. The converter 45 is electrically connected to the pulse generator 44 and is configured to divide a stimulation pulse signal of the pulse generator 44 into multiple channels of stimulation pulse signals, and each channel of stimulation pulse signal is released through one stimulation electrode 43. The pulse generator 44 is generally provided with two channel assemblies, and the two channel assemblies 12 are connected to two stimulation electrodes 43 to release two channels of pulse stimulation, however, the two channels of pulse stimulation cannot satisfy the therapeutic requirements, and the number of stimulation electrodes 43 needs to be increased. In this embodiment, multiple channel modules may be disposed on the converter 45, the channel module may be used for insertion of the insertion member 46, the converter 45 is provided so that one channel of multi-contact stimulation pulse may be separated into multiple channels of multi-contact stimulation pulses, and each channel of stimulation pulse signal is released through one stimulation electrode 43, thereby satisfying the requirements for more than two stimulation electrodes 43 for applying the electrical stimulation, and satisfying the clinical requirements for more electrode stimulation.

In a specific embodiment, the wire includes an extension wire 41 and multiple electrode wires 42. The converter 45 is electrically connected to the pulse generator 44 through the extension wire 41, and each stimulation electrode 43 is electrically connected to the converter 45 through the insertion member 46 on one electrode wire 42. The extension wire 41 and the multiple electrode wires 42 are provided so that the electrical connection between the pulse generator 44 and the multiple stimulation electrodes 43 is facilitated. The converter 45 may also be directly connected to the pulse generator 44.

Although the embodiments of the present application have been shown and described above, the above embodiments are exemplary and should not be construed as limiting the present application, variations, modifications, substitutions and changes to the above-described embodiments may be made within the range of the present application without departing from the principle and spirit of the present application, and all such changes shall fall within the scope of protection of the claims of the present application.

## Claims

1. A pulse generator, comprising:
a connection module, comprising a top cover and a channel assembly, wherein at least part of the channel assembly is disposed in the top cover and is configured for insertion of an insertion member;
a generator module, comprising a first housing, a circuit board and a battery connection assembly, wherein the connection module is mounted on the generator module, the circuit board is disposed in the first housing and is electrically connected to the channel assembly; and
a battery module, comprising a second housing and a battery located in the second housing, wherein the battery module is mounted on the generator module and is located outside the generator module, and the battery is electrically connected to the circuit board through the battery connection assembly.

2. The pulse generator of claim 1, wherein the top cover is an insulation member, the top cover comprises a front cover and a rear cover, the front cover and the rear cover are engaged to form an accommodation cavity, and at least one first channel groove is disposed on an inner side of the rear cover facing the front cover; and
the channel assembly comprises a channel cover and a plurality of connection members, a second channel groove corresponding to a first channel groove of the at least one first channel groove is formed on a side of the channel cover facing the rear cover, after the channel cover and the rear cover are engaged, the first channel groove and the second channel groove form an axial channel for insertion of the insertion member, a plurality of radial grooves are distributed on the axial channel at intervals, one connection member of the plurality of connection members which is configured to be electrically connected to the insertion member is disposed in each radial groove of the plurality of radial grooves, and the plurality of connection members are electrically connected to the circuit board.

3. The pulse generator of claim 2, wherein a first metal layer and a through hole communicating inside and outside of the channel cover are disposed on an inner wall of each radial groove of the plurality of radial grooves, an outer surface of the channel cover is provided with a plurality of second metal layers, each second metal layer of the plurality of second metal layers is electrically connected to the first metal layer in a radial groove corresponding to one through hole among the plurality of radial grooves through the one through hole, and the plurality of connection members are electrically connected to the circuit board through first metal layers and the plurality of second metal layers.

4. The pulse generator of claim 3, wherein the generator module further comprises a feedthrough member, the feedthrough member is an insulation member, a plurality of third metal layers are disposed on the feedthrough member, and each third metal layer of the plurality of third metal layers is electrically connected to one second metal layer of the plurality of second metal layers and the circuit board.

5. The pulse generator of claim 4, wherein the connection module further comprises a charging coil and an antenna, the charging coil is embedded in the front cover or the rear cover, the antenna is disposed on a side of the channel cover facing away from the plurality of radial grooves, a fourth metal layer and two fifth metal layers are disposed on the feedthrough member, the fourth metal layer is electrically connected to the antenna and the circuit board, the two fifth metal layers are electrically connected to the charging coil and the circuit board, respectively, and the fourth metal layer and the two fifth metal layers are close to two opposite ends of the feedthrough member, respectively.

6. The pulse generator of claim 2, wherein a first clamping portion facing the top cover is disposed on the first housing, a second clamping portion is disposed on the front cover or the rear cover, and in a case where the connection module is mounted on the generator module, the first clamping portion is in clamping connection with the second clamping portion.

7. The pulse generator of claim 6, wherein a clamping groove is disposed on the first clamping portion, the second clamping portion is a protrusion which has an inclined surface and disposed on an inner surface of the rear cover, and in a case where the connection module is mounted on the generator module, the first clamping portion is clamped on the second clamping portion under a guiding action of the inclined surface of the second clamping portion.

8. The pulse generator of claim 2, wherein the front cover is provided with an aperture communicating the inside and outside of the accommodation cavity, and the aperture of the front cover is a notch located at an edge of the front cover or an aperture not extending to an edge of the front cover; and
a first auxiliary positioning portion is disposed on an edge of the rear cover facing the first housing, a second auxiliary positioning portion is disposed on the first housing and is engaged with the rear cover, and in a case where the connection module is mounted on the generator module, the first auxiliary positioning portion is engaged with the second auxiliary positioning portion.

9. The pulse generator of claim 8, wherein the first housing is provided with a tongue plate deepening into the accommodation cavity, the accommodation cavity is provided with an insulating encapsulation material for filling a gap in the accommodation cavity, the insulating encapsulation material blocks the aperture on the front cover, and the insulating encapsulation material wraps the tongue plate.

10. The pulse generator of claim 2, wherein the passage assembly further comprises a waterproof member and a positioning member, the waterproof member and the positioning member are sequentially disposed at an opening of an axial channel from outside to inside, each of the waterproof member and the positioning member is provided with a hole which is used for the insertion member to pass through and enter the axial channel, the positioning member is configured to fix the insertion member, and the plurality of connection members are each a coil spring.

11. The pulse generator of claim 1, wherein the connection module further comprises an identification member, the identification member is disposed in the top cover, the identification member is a metal member and has a hollow structure, and the hollow structure of the identification member forms an identification of the identification member.

12. The pulse generator of claim 1, wherein a thread hole is disposed on the top cover.

13. The pulse generator of claim 1, wherein the battery connection assembly comprises a positive connector and a negative connector, the battery module further comprises a positive terminal and a negative terminal, the positive terminal and the negative terminal are connected to the battery, respectively, the positive terminal is electrically connected to the positive connector, and the negative terminal is electrically connected to the negative connector; and
the generator module further comprises a temperature measuring device, and the temperature measuring device is electrically connected to the circuit board and is configured to measure a temperature of the battery.

14. The pulse generator of claim 1, wherein the first housing and the second housing are titanium shells and are welded together to form a sealed accommodation space, and the second housing also serves as a housing of the battery itself.

15. An assembling method for the pulse generator of any one of claims 1 to 14, and the assembling method comprises:
separately assembling the connection module, the generator module and the battery module;
assembling and connecting the first housing and the second housing so that the battery module is mounted on the generator module; and
the connection module is mounted on the generator module.

16. The assembling method of claim 15, further comprising:
assembling and connecting the first housing and the second housing through welding; and
injecting an insulating encapsulation material into the top cover to connect the top cover and the first housing.

17. The assembling method of claim 16, wherein the insulating encapsulation material is epoxy resin.

18. A stimulation system, comprising:
a stimulation electrode;
the pulse generator of any one of claims 1 to 14; and
a wire provided with an insertion member, wherein the insertion member is configured to be inserted into the channel assembly, and the pulse generator is electrically connected to the stimulation electrode through the wire.

19. The stimulation system of claim 18, further comprising a converter, wherein a plurality of stimulation electrodes are provided, the converter is electrically connected to the pulse generator and is configured to divide a stimulation pulse signal of the pulse generator into a plurality of channels of stimulation pulse signals, and each channel of stimulation pulse signal of the plurality of channels of stimulation pulse signals is released through one stimulation electrode of the plurality of stimulation electrodes.

20. The stimulation system of claim 19, wherein the wire comprises an extension wire and a plurality of electrode wires, the converter is electrically connected to the pulse generator through the extension wire, and each stimulation electrode of the plurality of stimulation electrodes is electrically connected to the converter through the insertion member on one electrode wire of the plurality of electrode wires.
